# EUROPEAN PATENT SPECIFICATION

(11) **EP 3 177 277 B1**
(45) Date of publication and mention of the grant of the patent: **11.03.2020**
(21) Application number: 15750347.5
(22) Date of filing: 06.08.2015
(51) Int. Cl.: A23L 33/105, A23L 33/135, A23L 33/10, A61K 35/745, A61K 35/747, A61K 31/047, A61P 3/12, A61P 9/10, A61P 25/28, A61P 27/02

(54) **MYO-INOSITOL AND PROBIOTICS AND USES**
MYO-INOSITOL UND PROBIOTEN UND DEREN VERWENDUNG
MYO-INOSITOL ET PROBIOTIQUES, ET LEUR UTILISATION

(30) Priority: 08.08.2014 EP 14180399; 08.08.2014 EP 14180403; 05.06.2015 EP 15170902
(43) Date of publication of application: 14.06.2017
(73) Proprietor: Société des Produits Nestlé S.A., 1800 Vevey (CH)
(72) Inventor: SILVA ZOLEZZI, Irma, CH-1084 Carrouge (CH); GODFREY, Keith Malcolm, Ashurst Hampshire SO40 7AP (GB); BAKER, Philip Newton, Auckland 1011 (NZ); CHONG, Yap Seng, Singapore 268431 (SG); MACE, Catherine, CH-1005 Lausanne (CH); MINEHIRA CASTELLI, Kaori, CH-1804 Corsier-sur-Vevey (CH)
(74) Representative: Gagliardi, Tatiana
(86) International application number: PCT/EP2015/068187
(87) International publication number: WO 2016/020488

(56) References cited:
- EP-A1- 2 011 506
- EP-A1- 2 452 573
- WO-A1-2013/076121
- JP-A- 2006 213 684
- US-A- 5 763 392
- US-A1- 2012 027 737
- MARINE L. CROZE ET AL: "Chronic treatment with myo-inositol reduces white adipose tissue accretion and improves insulin sensitivity in female mice", THE JOURNAL OF NUTRITIONAL BIOCHEMISTRY, vol. 24, no. 2, 1 February 2013 (2013-02-01), pages 457-466, XP055212044, ISSN: 0955-2863, DOI: 10.1016/j.jnutbio.2012.01.008
- ROGOZINSKA EWELINA ET AL: "Nutritional manipulation for the primary prevention of gestational diabetes mellitus: a meta-analysis of randomised studies.", PLOS ONE 2015, vol. 10, no. 2, 26 February 2015 (2015-02-26), page e0115526, XP055211940, ISSN: 1932-6203

## Description

### Field of the invention

The invention relates to myo-inositol and probiotics to minimise fat accretion and/or to treat or prevent excess weight or obesity in a subject and/or for use in improving insulin sensitivity and/or treating or preventing an impaired glucose tolerance (IGT) and/or type II diabetes and/or for preventing cardio vascular disease and/or a condition associated with a low insulin sensitivity and/or any of the foregoing in a subject.

### Background

Glucose is a key nutrient which gives vital energy to living cells in our organs. The glucose can be derived from food and also de novo synthesized by the liver and kidney during a fasting condition. During digestion carbohydrates from glucidic foods are transformed into glucose that is passed through the intestinal wall into the bloodstream. This influx of glucose into the bloodstream results in an increase in the blood glucose level.

The level of blood glucose is tightly regulated by a hormone, insulin. As the blood glucose level increases insulin is secreted by the pancreas. Insulin is the hormone responsible for bringing glucose to insulin-sensitive tissues such as liver, muscle and adipose tissues, thus lowering the blood glucose level in the blood. However, some subjects do not respond adequately to insulin. These subjects have an insulin resistance or a low insulin sensitivity. This may be because insulin receptors in the insulin-sensitive tissues do not respond adequately to insulin. In response to an inadequate response to insulin the pancreas may secrete more insulin to compensate. These subjects may be considered as having an impaired glucose tolerance (hereinafter IGT).

Eventually, the pancreas may fail to keep up with the body's increased need for insulin, leading to type II diabetes. According to the World Health Organization, at least 171 million people worldwide suffer from type II diabetes. Its incidence is increasing rapidly, and it is estimated that by 2030, this number will almost double. An IGT (also referred to as prediabetes) is even more common, and more than one out of three adults have been estimated "prediabetic" or as having an IGT in the United States.

An IGT and/or type II diabetes are associated with an increased risk of a variety of diseases for example Cardio Vascular Disease (hereinafter CVD). CVD accounts for approximately 20% of all annual worldwide deaths, and remains one of the leading causes of death in both the developed and developing world.

Dietary and lifestyle changes, including healthier dietary habits and increased exercise, can be very efficient in improving an IGT and in preventing type II diabetes, however, patient compliance can be problematic. Drugs such as biguanides and thiazolidinediones are available for improving insulin sensitivity and/or and IGT and/or type II diabetes. However, many of these have unwanted side effects. Moreover, there is no practice of giving such drugs to prevent an IGT.

Croze ML et al, J.Nutr. BioChem. 2013 ; 24 : 457-466 (XP55212044) discloses that myoinositol is a viable nutritional strategy for the prevention and/or treatment of Type II diabetes.
US5763392 discloses that a composition comprising myoinositol may treat diabetes.
RogozinskaE et al, PLoS ONE 2015; 10(2) : 1-21 (XP55211940) is a summary and review of interventions for gestational diabetes mellitus. Among other interventions, myo-inositol and probiotics LGG and BB12 are mentioned.

Accordingly, there is a need to find alternative ways to improve insulin sensitivity and to treat or prevent an IGT and/or type II diabetes, and/or to prevent a condition associated with any of the foregoing in a subject.

There is a well-established link between body fat deposition, obesity, insulin sensitivity, an impaired glucose tolerance and type II diabetes. Accordingly, minimising fat accretion and/or preventing or treating excess weight or obesity in a subject may improve insulin sensitivity and/or prevent and/or treat an impaired glucose tolerance and type II diabetes and/or prevent a condition associated therewith in a subject e.g. CVD.

The inventors have surprisingly found that the administration of a combination of myo-inositol and one or more probiotic to a subject may minimise fat accretion (fat storage), and increase lean muscle mass in said subject. The effectiveness of this combination of ingredients is surprisingly better than could be expected on the basis of the effects of these ingredients when used alone. This better effect may be more than additive. Further, it has also been found that the administration of a combination of myo-inositol and one or more probiotic to a subject may increase the amount of insulin in the pancreas of said subject. This may result in a an improved insulin response to a glucose challenge and in consequence may improve insulin sensitivity and/or to treat or prevent an IGT and/or type II diabetes and/or to prevent CVD and/or a condition associated with a low insulin sensitivity and/or any of the foregoing (IGT, type II diabetes and CVD) in a subject.

### Summary of the Invention

The invention is set out in the claims. Myo-inositol and a combination of the Lactobacillus rhamnosus GG (CGMCC 1.3724) and Bifidobacterium lactis BB-12 (CNCM I-3446) probiotics are used to minimise fat accretion and/or to prevent or treat excess weight or obesity and/or to treat or prevent an IGT and/or type II diabetes in a subject e.g. a mammal such as a human, cat, or dog. The administration of the combination of myo-inositol and probiotics is not in-utero or through breastfeeding.

A low insulin sensitivity and/or an IGT and/or type II diabetes and/or CVD are associated with an increased risk of a variety of conditions, for example strokes, circulatory problems that in extreme cases can lead to amputation, diabetic retinopathy, kidney failure, hearing loss, and impaired cognitive ability. By improving insulin sensitivity and/or treating or preventing type II diabetes and/or preventing CVD, myo-inositol and one or more probiotics may also be used to treat or prevent associated conditions such as those listed in a subject.

The combination of myo-inositol and the above probiotics can be particularly effective, at minimising fat accretion and/or preventing or treating excess weight or obesity and/or at improving insulin sensitivity and/or treating or preventing an IGT and/or type II diabetes and/or preventing CVD in a subject, when used in combination with one or more of vitamin B2, B6, B12 and D.

The myo-inositol and the probiotics optionally further combined with one or more of vitamin B2, B6, B12 and D, may be administered enterally to a subject.

The myo-inositol, and the probiotics and optionally one or more of vitamin B2, B6, B12 and D, may be administered or employed in any form suitable for ingestion by a subject e.g. in the form of a composition for example a nutritional product, a food product, a functional food product, a healthy ageing product, a dairy product, a nutritional supplement, a pharmaceutical formulation, a beverage product, or a pet food product.

Myo-inositol and the probiotics and optionally one or more of vitamin B2, B6, B12 and D, may be used in the manufacture of a medicament for use to minimise fat accretion and/or to prevent or treat excess weight or obesity and/or to improve insulin sensitivity and/or to treat or prevent an IGT and/or type II diabetes and/or prevent CVD or a condition associated with a low insulin sensitivity and/or any of the foregoing in a subject.

Myo-inositol and the probiotics and optionally one or more of vitamin B2, B6, B12 and D may be provided along with a label indicating dosage requirements in a kit for use to improve insulin sensitivity and/or to treat or prevent an IGT and/or type II diabetes and/or prevent CVD or a condition associated with a low insulin sensitivity and/or any of the foregoing in a subject.

### Drawings

FIG 1 is a chart illustrating the effect of a combination of myo-inositol and probiotics on the delta % fat mass of rats after 10 weeks.
Figure 2 is a chart illustrating the effect of a combination of myo-inositol and probiotics on the ratio of fat gain to weight gain in rats after 10 weeks.
Figure 3 is a chart illustrating the effect of a combination of myo-inositol and probiotics on the amount of insulin in the pancreas at day 19.5 of gestation in pregnant Goto Kakizaki rats.
Figure 4 is a chart illustrating the effect of a combination of myo-inositol and probiotics on the AUC insulin increase at day 16.5 of gestation in pregnant Goto Kakizaki rats.
Figure 5 is a chart illustrating the effect of a combination of myo-inositol and probiotics on SLC5A11 transcription in gastrocnemius.

### Detailed Description

In a first aspect of the present invention there is provided myo-inositol and a combination of Lactobacillus rhamnosus GG (CGMCC 1.3724) and Bifidobacterium lactis BB-12 (CNCM I-3446), for use to prevent or treat excess weight or obesity and/or to treat or prevent an IGT and/or type II diabetes in a subject. The administration is not in-utero or through breastfeeding.

The term "subject" as used herein refers to a mammal and more particularly a cat, a dog or a human. The human may be an adult, child or baby including a preterm baby.

In a particular embodiment the subject is not a mammal that is trying to get pregnant, pregnant, or lactating.

A mammal is considered overweight or obese if their body fat percentage is too high.

A human female is considered obese if their body fat % is greater than 37%.

A human male is considered overweight if their body fat % is 21 to 24%.

A human male is considered obese if their body fat % is greater than 24 %.

A human child may be considered overweight or obese depending in what percentile they fall.

A human child may for instance be considered overweight if they fall in the 85-95^{th} percentile for body fat.

A human child may for instance be considered obese if they fall in the 95^{th} percentile or above for body fat.

Body fat % can be measured by carrying out a bioelectrical impedance analysis (hereinafter BIA) on the subject, or by subjecting the subject to nuclear magnetic resonance (hereinafter NMR).

Whether or not a mammal has an IGT or has type II diabetes may be determined by measuring its fasting glucose and/or HbAlc concentration, or by carrying out an oral glucose tolerance test (OGTT). The skilled person will be familiar with these tests and the criteria for diagnosing an IGT or type II diabetes. For humans the criteria for diagnosing an IGT or type II diabetes has been set out by the Standards of medical care in diabetes (2014) from the American Diabetes Association. A human subject is considered as having an IGT (or prediabetic condition) if their fasting plasma-glucose concentration equates to 5.6 mmol/L or more, or if their fasting HbA1C level are between 5.7-6.4 %, or blood glucose level ranges between 7.8 -11.0 mmol/L 2 hours after a 75gram glucose drink. A human subject is considered as having type II diabetes if their fasting plasma-glucose concentration equates to 7.0 mmol/L or more, or fasting HbAlc is higher than 6.5 %, or higherthan 11.1 mmol/L 2 hours after a 75gram glucose drink.

The term "treat" as used herein also encompasses amelioration and/or alleviation of a condition i.e. the amelioration and/or alleviation of the symptoms of a condition. It may for example encompass the reduction of the severity of a condition in a subject.

The term "prevent" as used herein refers to the prevention of the occurrence, or reduction of the risk of the occurrence, of a condition in a subject.

Any source or form of myo-inositol suitable for ingestion by a subject may be used in the invention.

The term myo-inositol as used herein refers to myo-inositol (*cis-*1, 2,3,5*-trans*-4,6-cyclohexanehexol) and /or a metabolite thereof.
The term metabolite as used herein refers to any substance produced and/or formed by the body of a subject from a particular compound after its administration e.g. ingestion. It includes active forms and catabolites of a compound.

A metabolite of myo-inositol can be selected from the group consisting of D-chiro-inositol and L-chiro-inositol. In particular the metabolite is D-chiro-inositol.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

The combination of probiotic bacteria comprises as first component Lactobacillus rhamnosus ATCC 53103 obtainable inter alia from Valio Oy of Finland under the trade mark LGG and Lactobacillus rhamnosus deposited by Nestle R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. The second component is Bifidobacterium lactis deposited at the Collection Nationale de Cultures De Microorganismes (CNCM)as CNCM 1-3446, sold inter alia by the Christian Hansen company of Denmark under the trade mark Bb12.

The combination of probiotics is a mixture of Lactobacillus rhamnosus CGMCC 1.3724 and Bifidobacterium lactis CNCM I-3446

The Lactobacillus rhamnosus CGMCC 1.3724 and Bifidobacterium lactis CNCM I-3446 can be used in a ratio of 1:99 to 99:1, however, more particularly they will be used in a ratio of 1:2 to 2 to 1, even more particularly 1:1.

The combination of myo-inositol and the probiotics may be particularly effective at minimizing fat accretion and/or preventing or treating excess weight or obesity and/or at improving insulin sensitivity and/or treating or preventing an IGT and/or type II diabetes and/or preventing CVD and/or a condition associated with a low insulin sensitivity and/or any of the forgoing in a subject, when further combined with one or more of vitamin B2, B6, B12 and D. The combination of myo-inositol and one or more probiotic when further combined with one or more of these listed ingredients may provide an improved effect over what could be expected on the basis of the effect of the myo-inositol and one or more probiotics, or one or more of vitamins B2, B6, B12 and D when used alone to minimise fat accretion and/or prevent or treat excess weight or obesity and/or to improve insulin sensitivity and/or to treat or prevent an IGT and/or type II diabetes and/or to prevent CVD and/or a condition associated with a low insulin sensitivity and/or any of the forgoing in a subject.

Accordingly, in an embodiment the combination of myo-inositol and the probiotics is further combined with one or more of vitamin B2, B6, B12 and D.

In particular the myo-inositol and one or more probiotic is further combined with vitamin B2, B6, B12 and D.

Any source or form of vitamins B2, B6, B12 and D suitable for ingestion by a subject may be used in the invention.

The term vitamin B2 as used herein refers to vitamin B2 and /or a metabolite thereof.

Any source or form of Vitamin B2 suitable for ingestion by a subject may be used. In particular vitamin B2 may be riboflavin e.g. riboflavin sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

The term vitamin B12 as used herein refers to vitamin B12 and /or a metabolite thereof.
The term vitamin B6 as used herein refers to vitamin B6 and /or a metabolite thereof.
The term vitamin D as used herein refers to vitamin D, a precursor thereof, and /or a metabolite thereof.

The term precursor as used herein refers to any substance administered e.g. ingested by a subject and used by the body of said subject to produced and/or form a particular compound.

In particular, the vitamin B6 may be pyroxidine hydrochloride, and/or a metabolite of vitamin B6 selected from the group consisting of Pyridoxal 5'-phosphate (hereinafter PLP).

In particular, the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, and 1,25-dihydroxyvitamin D2, and a combination of thereof.

More particularly the vitamin D is vitamin D3, a metabolite of vitamin D selected from the group consisting of 25-hydroxyvitamin D3, 1,25-dihydroxyvitamin, and combination thereof. Even more particularly the vitamin D3 is Cholecalciferol.

The myo-inositol and one or more probiotic, optionally further combined with one or more of vitamin B2, B6, B12 and D, can be employed in any effective dose that provides a benefit with respect to minimising fat accretion and/or preventing or treating excess weight or obesity and/or with respect to improving insulin sensitivity and/or the treatment or prevention of an IGT and/or type II diabetes.

With respect to fat accretion, excess weight and obesity, an effective dose may be any dose that lowers fat accretion in comparison to when said dose is not administered.

With respect to improving insulin sensitivity and/or an IGT and/or type II diabetes, an effective dose may be any dose that improves, by any degree, insulin sensitivity and/or an IGT and/or type II diabetes in a subject.

It is well within the purview of the skilled person to determine an effective dose.

With respect to minimising fat accretion, excess weight and/or obesity an effective dose may, for example, be determined by carrying out a bioelectrical impedance analysis (hereinafter BIA) on the subject, or by subjecting the subject to nuclear magnetic resonance (hereinafter NMR), before and after taking said dose for a certain period of time. The period may be 10 days to 3 months.

With respect to improving insulin sensitivity and/or an IGT and/or type II diabetes, an effective dose may, for example, be determined by testing the effect of a dose on a subject's fasting glucose plasma concentration, or fasting HbAlc concentration, or by carrying out an OGTT. An effective dose should improve a subject's fasting glucose plasma concentration and/or HbAlc concentration, and/or lower a subject's glycemic response.

Typically, an effective dose will depend on the type, age, size and health status of the subject, on the subject's lifestyle, as well as on its genetic heritage.

A particularly useful dose of myo-inositol may be from 0.2 to 5mg, 1.5 to 5mg, 2 to 4g, or 2g.
A particularly useful dose of vitamin B2, if present, may be 0.14 to 14 mg, 1 to 2.5mg, 1.5 to 2mg, or 1.8mg.
A particularly useful dose of the one or more probiotic, if present, is from 10e5 to 10e12 colony forming units (cfu), or 10e7 to 10e11 cfu.
A particularly useful dose of vitamin B6, if present, is 0.19 to 19 mg, or 2.6mg. A particularly useful dose of vitamin B12, if present, is 0.26 to 26µg, or 5.2µg.
A particularly useful dose of vitamin D, if present, is 1.5 to 100 µg, or 10µg.

The term "dose" as used herein refers to a daily quantity that is administered to a subject. The daily quantity or dose may be administered all at once or it may be spread out over several administrations throughout a day. The dose may be by administered by any known method, in particularly enterally e.g. orally.

The dose(s) may be administered at any time e.g. day time or night time. The dose may be particularly effective if administered before the subject has food or beverage e.g. has a meal or a snack.

In an embodiment the dose is spread over 2 administrations, in a particular the dose is spread over 2 administrations, one in the morning and one in the evening, in particularly before breakfast and before the evening meal.

The term "breakfast" as used herein refers to the first meal of the day.

The term "evening meal" as used herein refers the last meal of the day.

In a particular embodiment the administration is not in-utero or through breastfeeding.

There is also disclosed a kit for for use to minimise fat accretion and/or to prevent or treat excess weight or obesity and/or to improve glucose sensitivity and/or to treat or prevent an IGT and/or type II diabetes and/or to prevent CVD and/or a condition associated with a low insulin sensitivity and/or any of the foregoing in a subject, the kit comprising:
a. myo-inositol and one or more probiotic and optionally one or more of vitamin B2, B6, B12 and D
b. a label indicating dosage requirements for a subject.

The dosage requirements may be 2 administrations per day, in particular 2 administrations per day before any food or beverage. More particularly, 2 administrations per day wherein one is in the morning and one is in the evening. Even more particularly, 2 administrations per day wherein one is in the morning before breakfast and one is in the evening before the evening meal.

The myo-inositol and the probiotics, and the optional one or more of vitamin B2, B6, B12 and D may all be provided in a format providing sustained release of said vitamins or one or more probiotic. This way, these compounds can be consumed less frequently, while the body is still constantly supplied with sufficient amount them.

The myo-inositol and the probiotics, and optionally one or more of vitamin B2, B6, B12 and D, may be administered simultaneously e.g. in the same composition. Alternatively, said myo-inositol and/or one or more probiotic and/or one or more of the optional vitamin B2, B6, B12 and D may be administered separately to one another e.g. Myo-inositol (optionally with one or more of vitamin B2, B6, B12 and D) may be administered separately to the probiotics. The separate administration may be sequential or simultaneously but separate administration e.g. myo-inositol(optionally with one or more of vitamin B2, B6, B12 and D) may be administered at the same time but separately to said probiotics e.g. in two tablets. The myo-inositol(optionally with one or more of vitamin B2, B6, B12 and D) may for example be administered separately and consecutively e.g. in quick succession e.g. within 5, 4, 3, 2, 1 minute, of said probiotics.

Myo-inositol and the probiotics, and optionally one or more of vitamin B2, B6, B12 and D, and any other optional ingredients as set out herein, may be administered or employed in any form suitable for ingestion by the subject. They may for example be employed in the form of a composition e.g. a nutritional product, a food product, a functional food product, a healthy ageing product, a dairy product, a nutritional supplement, a pharmaceutical formulation, a beverage product, a diet, or a pet food product.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual. The term "healthy ageing product" as used herein, can refer to a diet or nutritional supplement that is intended as a means to extend lifespan in an individual. Such a product may additionally contain antioxidants or other compounds such as oxytocin, insulin, human chorionic gonadotropin (hCG), erythropoietin (EPO), dietary fiber, plant sterols, PUFA, and combinations thereof.

The term "antioxidants" as used herein refers to molecules capable of slowing or preventing the oxidation of other molecules. Preferably, antioxidants are selected from: beta-carotene, vitamin C, vitamin E, selenium, carotenoids, coenzyme Q10, flavonoids, glutathione, lutein, lycopene, polyphenols, vitamin A, vitamin B1, vitamin B6, vitamin B12, vitamin C, vitamin D, vitamin E, zeaxanthin, lipoic acid, carnosine, N-acetylcysteine, or combinations thereof.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art. The term "beverage product" as used herein, refers to a nutritional product in liquid or semi-liquid form that may be safely consumed by an individual.

The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

Myo-inositol, in combination with the probiotics and optionally one or more of vitamin B2, B6, B12 and D, may also be used in combination with other vitamins and minerals. e.g. calcium, magnesium, phosphorus, iron, zinc, copper, iodine, selenium, vitamin A or retinol activity equivalent (RAE) e.g. beta carotene or a mix of carotenoids, vitamin C, vitamin B1, niacin, folic acid, biotin, vitamin E.

It may be particularly beneficial if myo-inositol the probiotics and optionally one or more of vitamin B2, B6, B12 and D, is used in combination with one or more of the following micronutrients in the following amounts: 100 to 2500 mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg Vitamin C, 0.14 to 14 mg Vitamin B1, 1.8 to 35 mg niacin, 60 to 1000 µg folic acid, 3 to 300 µg biotin, 1.9 to 109 µg Vitamin E.

Myo-inositol and the probiotics, and optionally one or more of vitamin B2, B6, B12 and D, may also be advantageously used in combination with at least one oligosaccharide and/or at least one long chain polyunsaturated fatty acids, such as arachidonic acid (ARA), eicosapentaenoic acid (EPA) and/or docosahexaenoic acid (DHA). The oligosaccharides and long chain polyunsaturated fatty acids can be used in any concentration safe for administration to the subject.

Myo-inositol and the probiotics and optionally one or more of vitamin B2, B6, B12 and D, may also be used in combination with other ingredients commonly used in the form of composition in which it is employed e.g powdered nutritional supplement, a food product, or a dairy product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavor agents, osmotic agents, pharmaceutically acceptable carriers, preservatives, sugars, sweeteners, texturizers, emulsifiers, water and any combination thereof.

However, notwithstanding the above, a high fat intake can be undesirable and is linked to an increased risk of excess weight and obesity. Accordingly, in a particular embodiment the myo-inositol and one or more probiotic and optionally one or more of vitamin B2, B6, B12 and D is not used in combination with a long chain polyunsaturated fatty acid and/or animal fats and/or vegetable fats.

In another particular embodiment the myo-inositol and the probiotics and optionally one or more of vitamin B2, B6, B12 and D is not used in combination with an oligosaccharide.

In a particular embodiment myo-inositol is used only in combination with the probiotics.

In a particular embodiment myo-inositol is used only in combination with the probiotics and one or more of vitamin B2, B6, B12 and D.

As stated hereinabove, a low insulin sensitivity and/or type II diabetes in a subject is associated with a variety of conditions that affect the subject e.g. CVD. A low insulin sensitivity and/or an IGT and/or type II diabetes is considered as being associated with a condition if it increases the risk of a subject having or developing that condition during its lifetime.

Non limiting examples of conditions associated with a low insulin sensitivity and/or type II diabetes include: CVD, strokes, circulatory problems that in extreme cases can lead to amputation, diabetic retinopathy, kidney failure, hearing loss, and impaired cognitive ability.

A low insulin sensitivity, an IGT, and type II diabetes are more prevalent amongst subjects who are overweight or obese, or if there is a family history of type II diabetes.

Accordingly, myo-inositol and the probiotics, and optionally one or more of vitamin B2, B6, B12 and D, may be particularly beneficial for improving a low insulin sensitivity and/or treating and/or preventing an IGT and/or type II diabetes and/ or preventing CVD and/or conditions associated with a low insulin sensitivity and/or any of the foregoing in a one or more of these subsets of subjects.

As would be clear to the skilled person, the invention provides a composition comprising myo-inositol and the probiotics, and optionally one or more of vitamin B2, B6, B12 and D, for use to prevent or treat excess weight or obesity and/or to treat or prevent an IGT and/or type II diabetes in a subject.

Further advantages and features of the present invention are apparent from the figure and non-limiting example.

The present invention will now be described in further details by the way of the following examples.

### Examples

### Example 1

An example composition comprising myo-inositol in combination with vitamins B2, B6, B12 and D, and Lactobacillus rhamnosus GG¹ and Bifidobacterium lactis BB12² is set out in table 1.

The composition in table I is for a nutritional supplement in a powder form, intended to be sprinkled on food.

**Table 1 : Composition of Example 1**

| **Ingredient** | **Amount per daily dose** |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG¹⁾ | 1x10⁹ cfu |
| Bifidobacterium lactis BB12²⁾ | 1x10⁹ cfu |

| | |
|---|---|
| 1) Strain deposited as CGMCC 1.3724 2) Strain deposited as CNCM I-3446 | |

The composition may be provided as a kit of parts comprising in one sachet the probiotic as a powder and in a second sachet all other ingredients.

### Example 2

A milk powder drink for a human subject to be reconstituted in water is provided consisting of 30g of milk powder per serving admixed with the ingredients listed in Table 1, in half of the amounts mentioned in Table 1. The composition is to be administered to a human subject twice per day.

### Example 3

80 Females virgin Goto Kakizaki (GK) of 4-5 weeks old with close body weight, were purchased from Charles River (France and USA), caged singly in a temperature-controlled room (22±1°C) with a 12-h light/dark cycle, and maintained on a AIN-93G growing rodent diet (Research diets, USA, Table 2).

After one week on acclimation, GK rats were divided into 4 groups (n=20) after a randomization based on AUC glucose value and body weight, and received their corresponding treatment for 10 weeks as below :
1. Group Control: GK female rats ate *ad-libitum* a AIN-93G diet + a control gelatin (Table 3).
2. Group Myo-inositol : GK female rats ate *ad-libitum* a AIN93-G diet supplemented with myo-inositol diet (1 g myo-inositol* for 100 g de diet) + a control gelatin (Table 3).
3. Group Probiotic : GK female rats ate *ad-libitum* a AIN93-G + a gelatin containing 10⁹ LPR CFU and 10⁹ B.lactis CFU(Table 3).
4. Group Mix : GK female ate *ad-libitum* a AIN93G diet supplemented with myo-inositol diet (1 g myo-inositol for 100g de diet) + a gelatin containing 10⁹ LPR CFU and 10⁹ B.lactis CFU (Table 3).
   *Kirsh Pharma

At 15 weeks of age, females were housed with Wistar males (Charles Rivers, France) until a vaginal plug was observed, indicating day 0.5 of gestation. Female rats remained on their allocated experimental diet throughout gestation. At 16.5 days of gestation an OGTT was performed on all females (n=20 per group) and at 19.5 days of gestation half of the animals (n=9-11 per group) were sacrificed after 6 hours fasting.

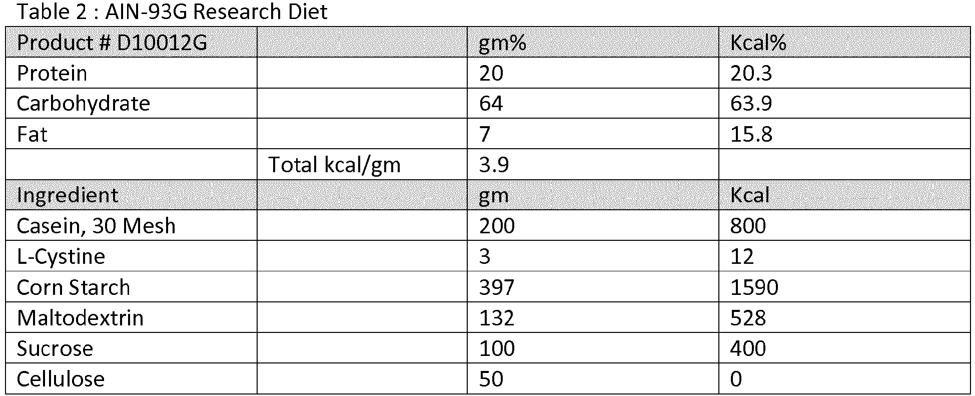

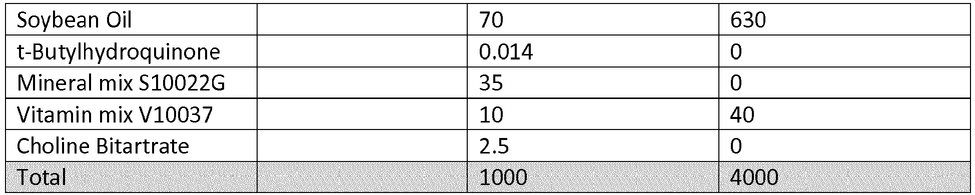

**Table 3 : Composition of the experimental Gelatin**

| | Treatment | Control |
|---|---|---|
| Gelatin (g) | 12 | 12 |
| Sacharine (mg) | 0.06 | 0.06 |
| Probiotic (g)* | 7.7 | 0 |
| Maltodextrin mix (g) | 0 | 6.7 |
| TS + Water (ml) | 80.2 | 81.2 |
| Total (ml) | 99.96 | 99.96 |

### Measurements and analysis

### Physiological measurements and sample collections:

- An oral glucose tolerance test (OGTT) was performed after 6 hours of day deprivation (from 7.30 am to 13.30 pm) in all animals at age of 5 weeks and 16.5 days of gestation. During OGTT two baseline blood samples were taken from the tail vein, with at least 10 minutes interval between sampling (time -10 and 0), followed by an oral gavage of glucose solution (20% wt/v) at dose of 1.5 g/Kg body weight. Then further blood samples were collected from tail incision at 15, 30, 45, 60, 90 and 120 min after glucose administration. All blood samples were analysed directly for blood glucose level, using Contour Next glucometers (Bayer AG, Zurich, Switzerland).
- The body weight and food intake of the animals were measured 2 times / week throughout the study.
- Body composition (body fat, lean mass and body water content) was measured before and after 10 weeks of treatment by using nuclear magnetic resonance (EchoMRI TM 2004, Echo Medical Systems, Houston, USA). From these data collected it was possible to calculate the delta %fat and the fat gain / weight gain ratio as following :
- The delta %fat: (% fat at the end of the treatment - % fat before the start of the treatment)
- Fat gain / weight gain : (fat (g) at the end of the treatment period - fat(g) before the start of the treatment) / (body weight (g) at the end of the treatment period - body weight (g) before the start of the treatment)
- lean gain / weight gain : (lean (g) at the end of the treatment period - lean (g) before the start of the treatment) / (body weight (g) at the end of the treatment period - body weight (g) before the start of the treatment)
- Fat gain : fat (g) at the end of the treatment period - fat(g) before the start of the treatment
- lean gain : lean (g) at the end of the treatment period - lean (g) before the start of the treatment
- % fat mass : fat mass (g) / body weight
- % lean mass : lean mass (g) / body weight

**Sacrifice** : dams were decapitated after 6 hours of day deprivation (from 7.30 am to 13.30 pm) and organ collected by dissection, weighted and immediately frozen in liquid nitrogen. They were kept at -80°C until analysis.
- Insulin pancreatic measurement : . Pancreas was weighted during the sacrifice and preserved in an acid-ethanol-H2O solution until insulin extraction For determination of pancreatic insulin, pancreas was homogenized with an acid-ethanol (solution v/v: 75% ethanol, 1.5% of 37% HCI and 23.5% distilled water) and incubated at -20°C overnight. The insulin content in the supernatant was measured by an ELISA method using kits from Crystal Chem. Inc (IL, USA).

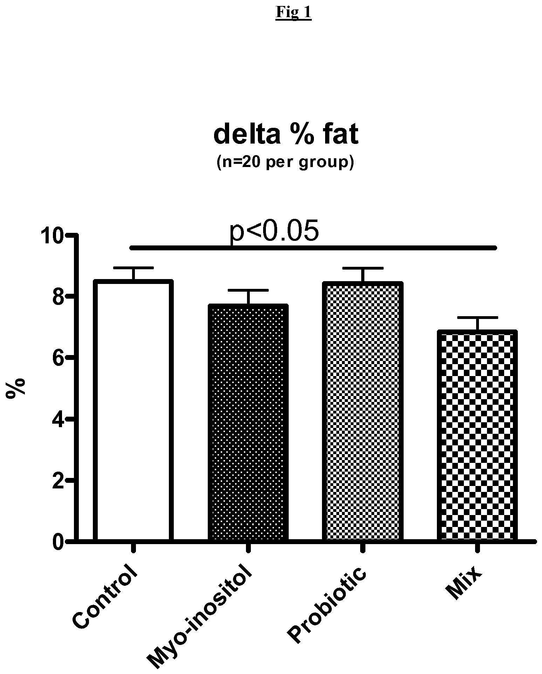

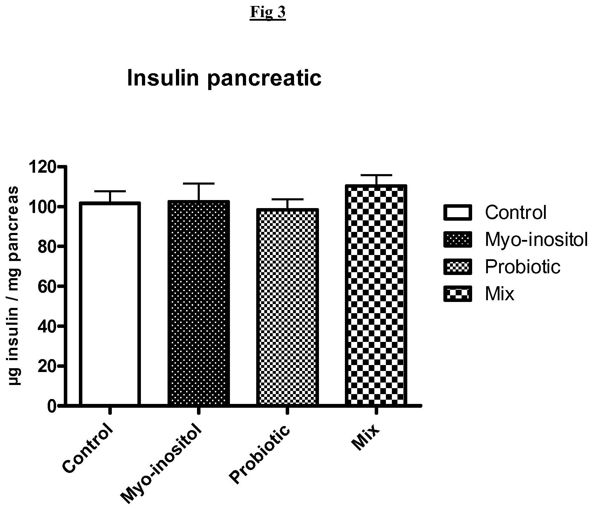

The mean value for each measurement is shown in table 4. Results for delta % fat, fat gain/ weight gain, µg insulin pancreas/ g pancreas can also be seen in Figures 1 to 3.

**Table 4**

| | Control | Myo-inositol | Probiotics | Mix (myo-inositol+probiotics) |
|---|---|---|---|---|
| % fat mass | 19.5 | 18.7 | 19.3 | 18.1 |
| % lean mass | 66.4 | 67.8 | 67.3 | 68.3 |
| Delta % fat | 8.8 | 7.7 | 8.4 | 6.8 |
| Fat gain/ weight gain | 0.28 | 0.27 | 0.27 | 0.24 |
| Lean gain/ weight gain | 0.57 | 0.58 | 0.58 | 0.60 |
| % fat gain | 27.6 | 26.6 | 27.3 | 24.7 |
| % lean gain | 57.4 | 58.3 | 58.5 | 60.3 |
| Body weight gain | 107 | 100 | 105 | 102 |
| Fat gain (g) | 29.8 | 26.8 | 28.8 | 25.3 |
| Lean gain (g) | 61.7 | 58.5 | 61.3 | 61.5 |
| µg insulin pancreas/ g pancreas | 101 | 101 | 98.5 | 110.3 |
| AUC insulin increase | 2 | 5 | -2 | 13 |
| FGIR | 3.44 | 3.42 | 3.24 | 4.21 |
| HOMA1-IR | 10.42 | 9.29 | 9.76 | 9.07 |

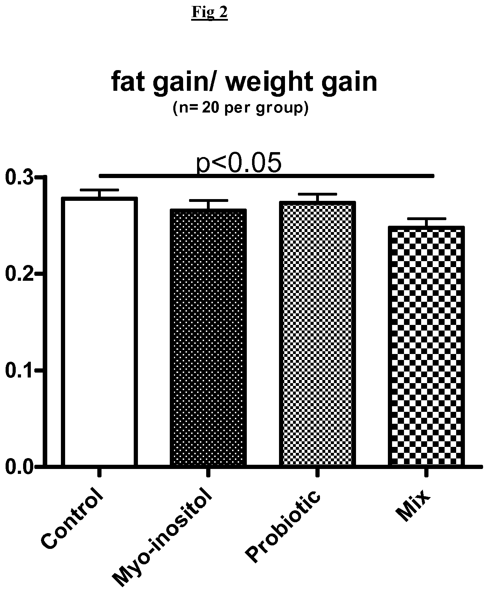

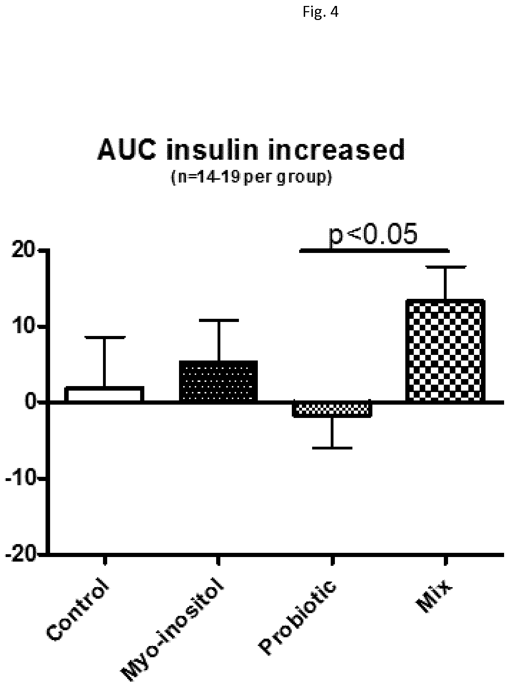

The mean value for each measurement is shown in table 11. Results can also be seen in Figures 2 to 12.

### Example 4

Measurement of how supplementation of myoinositol and probiotics affects transcription of SLC5A11 in rat gastrocnemius during pregnancy.

Gene expression analysis was performed on muscle (gastrocnemius) tissue samples of 10 dams of each group described in example 4. Rats were sacrificed under anesthesia (isofluarane) at 19.5 days of gestation after 6 hours fasting. This analysis was done using the GeneChip® Rat Genome 230 2.0 Array that provides comprehensive coverage of the transcribed rat genome using more than 31,000 probe sets, analyzing over 30,000 transcripts and variants from over 28,000 well-substantiated rat genes.

The steps performed for each sample are the following:

### 1) Sample preparation for microarrays analysis.

First, the tissue was pulverized using a Covaris cryoPREP™ CP02 (Covaris Ltd), and then 10-20 mg of powder were transfered to tubes frozen in liquid nitrogen to avoid thawing the samples. Then, lysis and extraction was done using the FastPrep and Agencourt RNAdvance Tissue Kit protocol (vers. Jan 2015). Once extracted RNA was quantified using the Quant-it Quant-iT™ RiboGreen® RNA Reagent and Kit, and the quality control was performed using the Standard Sensitivity RNA analysis Kit and the Fragment Analyzer (Advanced Analytical Technologies, Inc). All samples with a RQN>7.5 were eligible for cRNA synthesis. cRNA synthesis was performed using the Illumina® TotalPrep™-96 RNA Amplification Kit, starting with 300ng of Total RNA in 9µL Nuclease-free water, and performing a modification to the commercial protocol to use the Agencourt RNAclean XP kit for the purification steps instead of using the two purifications steps of the Illumina TotalPrep-96 kit. cRNA was then quantified and the Quality Control performed using the Quant-it Quant-iT™ RiboGreen® RNA Reagent and Kit and the Standard Sensitivity RNA analysis Kit, respectively. The Fragment Analyzer (Advanced Analytical Technologies, Inc) was used and the expected average size should be of 1580nt or greater. Finally, the cRNA was fragmented using a 5X Array Fragmentation Buffer (Affymetrix Kit IVT) and samples were incubated for 35 min at 94°C.Finally, the hybridization was prepared, first a mastermix was generated containing per sample 37µLof Control Oligo B2 (3 nM), 11µL of 20X Hybridization Controls (must be heated 5 min at 65°C before), 110µL of 2X Hybridization Mix, 22µL of DMSO and 43.9µl of Nuclease-free Water. For each sample 29.4µL fragmented cRNA + 190.6µL hybridization mix were added in a 250 µL PCR plate, ad these hybridization cocktails were kept at -20°C until use or -80°C if use is not in the same week.

### 2) Microarrays Hybridization.

For this step the GeneChip® Hybridization Wash and Stain Kit (part nr: 900720, Affymetrix, Inc.) was used. First, the Hybridization Cocktail was heated to 99 °C for 5 minutes and 45°C for another 5 min in a PCR machine. Meanwhile, per array (Rat Genome 230 2.0 Array,2pk GeneChip, part nr: 900505, Affymetrix, Inc.) 200µL of Pre-Hybridization Mix were used to fill the probe array and then proceed to incubate at 45 °C in the oven (Affymetrix Hybridization Oven 645, Affymetrix, Inc) for 10 minutes with rotation. Then, the array was removed from the hybridization oven and the pre-hyb solution removed to then be refilled with 200µL of Hybridization Cocktail. Finally, the probe array was placed in the hybridization oven, set to 45 °C and it was hybridized for 16 hours.

### 3) Microarrays Staining and scanning

For this the GeneChip® Expression Wash, Stain and Scan protocol was strictly followed as described by the manufacturer. Arrays were processed using the Affymetrix Fluidic Station 450, the Affymetrix GeneChip Autoloader and the Affymetrix GeneChip Scanner. The gene expression levels data were extracted and analyzes using a workflow from a dedicated commercial software (Partek®)

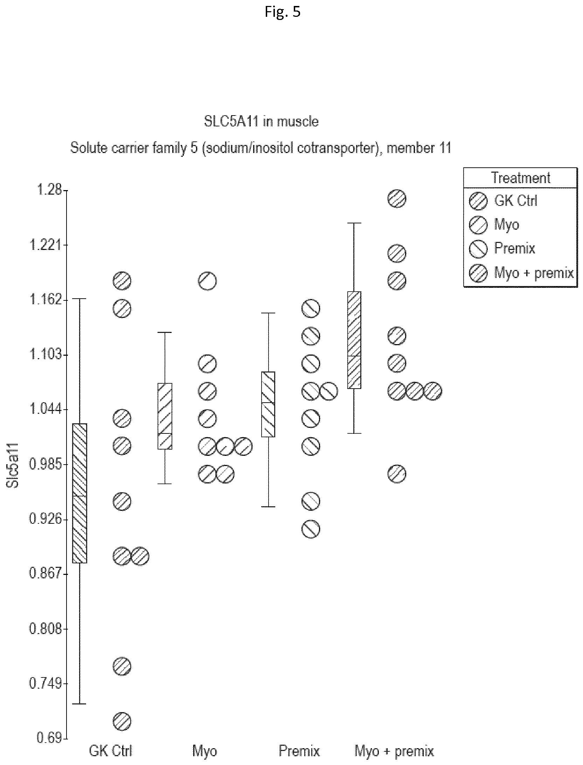

Results are shown in Figure 5

**Print Out (Original in Electronic Form)**

| (This sheet is not part of and does not count as a sheet of the international application) | | |
|---|---|---|
| **0-1** | **Form PCT/RO/134** | |
| | **Indications Relating to Deposited Microorganism(s) or Other Biological Material (PCT Rule 13bis)** | |
| 0-1-1 | Prepared Using | **PCT Online Filing** |
| | | **Version 3.5.000.244e MT/FOP 20141031/0.20.5.20** |
| **0-2** | **International Application No.** | |
| **0-3** | **Applicant's or agent's file reference** | 14729-WO-PCT |
| | | |
| **1** | **The indications made below relate to the deposited microorganism(s) or other biological material referred to in the description on:** | |
| **1-1** | **Paragraph number** | |
| **1-3** | **Identification of deposit** | |
| 1-3-1 | Name of depositary institution | **CNCM Collection nationale de cultures de microorganismes (CNCM)** |
| 1-3-2 | Address of depositary institution | **Institut Pasteur, 28, Rue du Docteur Roux, 75724 Paris Cedex 15, France** |
| 1-3-3 | Date of deposit | **07 June 2005 (07.06.2005)** |
| 1-3-4 | Accession Number | **CNCM I-3446** |
| **1-5** | **Designated States for Which Indications are Made** | **All designations** |
| **2** | **The indications made below relate to the deposited microorganism(s) or other biological material referred to in the description on:** | |
| **2-1** | **Paragraph number** | |
| **2-3** | **Identification of deposit** | |
| 2-3-1 | Name of depositary institution | **CGMCC China General Microbiological Culture Collection Center (CGMCC) Institute of Microbiology, Chinese Academy of Sciences, No. 1 West Beichen Road, Chaoyang District, Beijing 100 101, China** |
| 2-3-2 | Address of depositary institution | |
| 2-3-3 | Date of deposit | **01 October 2004 (01.10.2004)** |
| 2-3-4 | Accession Number | **CGMCC 1.3724** |
| **2-5** | **Designated States for Which Indications are Made** | **All designations** |

| **FOR RECEIVING OFFICE USE ONLY** | | |
|---|---|---|
| **0-4** | **This form was received with the international application:** (yes or no) | YES |
| 0-4-1 | Authorized officer | Hurenkamp, Jaap |

| **FOR INTERNATIONAL BUREAU USE ONLY** | | |
|---|---|---|
| **0-5** | **This form was received by the** | |

## Claims

1. A composition comprising myo-inositol and one or more probiotic for use in the prevention of excess weight or obesity and/or to treat or prevent an impaired glucose tolerance and/or type II diabetes wherein the one or more probiotic is a combination of lactobacillus rhamnosus GG (CGMCC 1.3724) and bifidobacterium lactis BB-12 (CNCM 1-3446, and wherein administration is not in-utero or through breastfeeding.

2. A composition for use in accordance with claim 1, wherein said composition further comprises one or more of vitamin B2, B6, B12 and D.

3. A composition for use according to any one of claims 1 to 2, wherein myo-inositol is used in a concentration of 0.2 to 5 mg per daily dose, and the one or more probiotic is used in a concentration of 10e5 to 10e12 colony forming units (cfu) per daily dose.

4. A composition for use according to any one of claims 2 to 3, wherein vitamin B2 if used is used in a concentration of 0.14 to 14 mg per daily dose, vitamin B6 if used is used in a concentration of 0.14 to 14 mg per daily dose, B12 if used is used in a concentration of 0.26 to 26 µg per daily dose, vitamin D if used is used in a concentration of 1.5 to 100 µg per daily dose.

5. A composition for use in accordance with any one of claims 1 to 4, wherein the subject is a mammal selected from the group consisting of; a cat, a dog, a human.

6. A composition for use in accordance with any one of claims 1 to 5, wherein said composition is administrated enterally, more preferably orally and preferably in the form of a nutritional product, a food product, a functional food product, a healthy ageing product, a dairy product, a nutritional supplement, a pharmaceutical formulation, a beverage product, a diet, or a pet food product.

7. Myo-inositol and one or more probiotic for use in the prevention of excess weight or obesity and/or in the treatment or prevention of an impaired glucose tolerance and/or type II diabetes in a subject, wherein said myo-inositol and one or more probiotic are administered simultaneously, separately or sequentially, wherein the one or more probiotic is a combination of lactobacillus rhamnosus GG (CGMCC 1.3724) and bifidobacterium lactis BB-12 (CNCM I-3446), and wherein administration is not in-utero or through breastfeeding

8. Myo-inositol and one or more probiotic for use according to claim 7 wherein said myo-inositol and one or more probiotic are used with one or more of vitamin B2, B6, B12 and D and wherein said myo-inositol and/or one or more probiotic are optionally administrated separately or sequential to one or more of vitamin B2, B6, B12 and D.

9. Myo-inositol and one or more probiotic for use according to claims 7 or 8, wherein myo-inositol is used in a concentration of 0.2 to 5 mg per daily dose and one or more probiotic is used in a concentration of 10e5 to 10e12 colony forming units (cfu) per daily dose.

10. Myo-inositol and one or more probiotic for use according to any one of claims 7 to 9, wherein vitamin B2 if used is used in a concentration of 0.14 to 14 mg per daily dose, vitamin B6 if used is used in a concentration of 0.14 to 14 mg per daily dose, B12 if used is used in a concentration of 0.26 to 26 µg per daily dose, vitamin D if used is used in a concentration of 1.5 to 100 µg per daily dose

11. Myo-inositol and one or more probiotic for use according to any one of claims 7 to 10, wherein wherein the subject is a mammal selected from the group consisting of; a cat, a dog, a human, and wherein said myo-inositol is preferably administrated enterally, more preferably orally and preferably in the form of a nutritional product, a food product, a functional food product, a healthy ageing product, a dairy product, a nutritional supplement, a pharmaceutical formulation, a beverage product, a diet, or a pet food product.

## Patentansprüche

1. Zusammensetzung, umfassend Myo-Inositol und ein oder mehrere Probiotika zur Verwendung bei der Verhinderung von Übergewicht oder Fettleibigkeit und/oder zum Behandeln oder Verhindern einer eingeschränkten Glucosetoleranz und/oder eines Typ-II-Diabetes, wobei das eine oder die mehreren Probiotika eine Kombination aus Lactobacillus rhamnosus GG (CGMCC 1.3724) und Bifidobacterium lactis BB-12 (CNCM I-3446) ist und wobei eine Verabreichung nicht im Uterus oder durch Stillen erfolgt.

2. Zusammensetzung zur Verwendung nach Anspruch 1, wobei die Zusammensetzung ferner ein oder mehrere von Vitamin B2, B6, B12 und D umfasst.

3. Zusammensetzung zur Verwendung nach einem der Ansprüche 1 bis 2, wobei Myo-Inositol in einer Konzentration von 0,2 bis 5 mg pro Tagesdosis verwendet wird und das eine oder die mehreren Probiotika in einer Konzentration von 10e5 bis 10e12 koloniebildenden Einheiten (kbE) pro Tagesdosis verwendet werden.

4. Zusammensetzung zur Verwendung nach einem der Ansprüche 2 bis 3, wobei Vitamin B2, wenn verwendet, in einer Konzentration von 0,14 bis 14 mg pro Tagesdosis verwendet wird, Vitamin B6, wenn verwendet, in einer Konzentration von 0,14 bis 14 mg pro Tagesdosis verwendet wird, B12, wenn verwendet, in einer Konzentration von 0,26 bis 26 µg pro Tagesdosis verwendet wird, Vitamin D, wenn verwendet, in einer Konzentration von 1,5 bis 100 µg pro Tagesdosis verwendet wird.

5. Zusammensetzung zur Verwendung in Übereinstimmung mit einem der Ansprüche 1 bis 4, wobei der Proband ein Säuger ist, ausgewählt aus der Gruppe, bestehend aus; einer Katze, einem Hund, einem Menschen.

6. Zusammensetzung zur Verwendung in Übereinstimmung mit einem der Ansprüche 1 bis 5, wobei die Zusammensetzung enterisch verabreicht wird, mehr bevorzugt oral und vorzugsweise in der Form eines Nahrungsmittelprodukts, eines Lebensmittelprodukts, eines funktionellen Lebensmittelprodukts, eines Produkts zum gesunden Altern, eines Milchprodukts, einer Nahrungsmittelergänzung, einer pharmazeutischen Formulierung, eines Getränkeprodukts, einer Diät oder eines Haustierfutterprodukts.

7. Myo-Inositol und ein oder mehrere Probiotika zur Verwendung bei der Verhinderung von Übergewicht oder Fettleibigkeit und/oder bei der Behandlung oder Verhinderung einer eingeschränkten Glucosetoleranz und/oder eines Typ-II-Diabetes bei einem Probanden, wobei das Myo-Inositol und das eine oder die mehreren Probiotika gleichzeitig, separat oder nacheinander verabreicht werden, wobei das eine oder die mehreren Probiotika eine Kombination aus Lactobacillus rhamnosus GG (CGMCC 1.3724) und Bifidobacterium lactis BB-12 (CNCM I-3446) ist und wobei eine Verabreichung nicht im Uterus oder durch Stillen erfolgt.

8. Myo-Inositol und ein oder mehrere Probiotika zur Verwendung
nach Anspruch 7, wobei das Myo-Inositol und das eine oder die mehreren Probiotika mit einem oder mehreren von Vitamin B2, B6, B12 und D verwendet werden und wobei das Myo-Inositol und/oder das eine oder die mehreren Probiotika wahlweise separat oder nach einem oder mehreren von Vitamin B2, B6, B12 und D verabreicht werden.

9. Myo-Inositol und ein oder mehrere Probiotika zur Verwendung nach einem der Ansprüche 7 oder 8, wobei Myo-Inositol in einer Konzentration von 0,2 bis 5 mg pro Tagesdosis verwendet wird und ein oder mehrere Probiotika in einer Konzentration von 10e5 bis 10e12 koloniebildenden Einheiten (kbE) pro Tagesdosis verwendet werden.

10. Myo-Inositol und ein oder mehrere Probiotika zur Verwendung nach einem der Ansprüche 7 bis 9, wobei Vitamin B2, wenn verwendet, in einer Konzentration von 0,14 bis 14 mg pro Tagesdosis verwendet wird, Vitamin B6, wenn verwendet, in einer Konzentration von 0,14 bis 14 mg pro Tagesdosis verwendet wird, B12, wenn verwendet, in einer Konzentration von 0,26 bis 26 µg pro Tagesdosis verwendet wird, Vitamin D, wenn verwendet, in einer Konzentration von 1,5 bis 100 µg pro Tagesdosis verwendet wird.

11. Myo-Inositol und ein oder mehrere Probiotika zur Verwendung nach einem der Ansprüche 7 bis 10, wobei
wobei der Proband ein Säuger ist, ausgewählt aus der Gruppe, bestehend aus; einer Katze, einem Hund, einem Menschen, und wobei das Myo-Inositol vorzugsweise enterisch verabreicht wird, mehr bevorzugt oral und vorzugsweise in der Form eines Nahrungsmittelprodukts, eines Lebensmittelprodukts, eines funktionellen Lebensmittelprodukts, eines Produkts zum gesunden Altern, eines Milchprodukts, einer Nahrungsmittelergänzung, einer pharmazeutischen Formulierung, eines Getränkeprodukts, einer Diät oder eines Haustierfutterprodukts.

## Revendications

1. Composition comprenant du myo-inositol et un ou plusieurs probiotiques pour une utilisation dans la prévention de la surcharge pondérale ou de l'obésité et/ou pour traiter ou prévenir un trouble de la tolérance au glucose et/ou le diabète de type II, le ou les probiotiques étant une combinaison de lactobacillus rhamnosus GG (CGCCM 1.3724) et de bifidobacterium lactis BB-12 (CNCM 1-3446, et l'administration n'étant pas in-utero ou par l'allaitement.

2. Composition pour une utilisation selon la revendication 1, ladite composition comprenant en outre une ou plusieurs des vitamines B2, B6, B12 et D.

3. Composition pour une utilisation selon l'une quelconque des revendications 1 à 2, dans laquelle le myo-inositol est utilisé à une concentration de 0,2 à 5 mg par dose journalière, et dans laquelle le ou les probiotiques sont utilisés à une concentration de 10e5 à 10e12 unités formant colonies (ufc) par dose journalière.

4. Composition pour une utilisation selon l'une quelconque des revendications 2 à 3, dans laquelle la vitamine B2, si elle est utilisée, est utilisée à une concentration de 0,14 à 14 mg par dose journalière, la vitamine B6, si elle est utilisée, est utilisée à une concentration de 0,14 à 14 mg par dose journalière, la vitamine B12, si elle est utilisée, est utilisée à une concentration de 0,26 à 26 µg par dose journalière, la vitamine D, si elle est utilisée, est utilisée à une concentration de 1,5 à 100 µg par dose journalière.

5. Composition pour une utilisation selon l'une quelconque des revendications 1 à 4, le sujet étant un mammifère choisi dans le groupe constitué par ; un chat, un chien, un être humain.

6. Composition pour une utilisation selon l'une quelconque des revendications 1 à 5, ladite composition étant administrée par voie entérale, plus préférablement par voie orale et de préférence sous la forme d'un produit nutritionnel, d'un produit alimentaire, d'un produit alimentaire fonctionnel, d'un produit pour un vieillissement sain, d'un produit laitier, d'un complément nutritionnel, d'une formulation pharmaceutique, d'un produit de boisson, d'un régime alimentaire, ou d'un produit alimentaire pour animal domestique.

7. Myo-inositol et un ou plusieurs probiotiques pour une utilisation dans la prévention de la surcharge pondérale ou de l'obésité et/ou dans le traitement ou la prévention d'un trouble de la tolérance au glucose et/ou du diabète de type II chez un sujet, ledit myo-inositol et un ou des probiotiques étant administrés simultanément, séparément ou séquentiellement, le ou les probiotiques étant une combinaison de lactobacillus rhamnosus GG (CGCCM 1.3724) et de bifidobacterium lactis BB-12 (CNCM I-3446), et l'administration n'étant pas in-utero ou par l'allaitement

8. Myo-inositol et un ou plusieurs probiotiques pour une utilisation selon la revendication 7, le myo-inositol et le ou les probiotiques étant utilisés avec une ou plusieurs vitamines B2, B6, B12 et D, et ledit myo-inositol et/ou un ou des probiotiques étant facultativement administrés séparément ou séquentiellement par rapport à une ou plusieurs des vitamines B2, B6, B12 et D.

9. Myo-inositol et un ou plusieurs probiotiques pour une utilisation selon les revendications 7 ou 8, le myo-inositol étant utilisé à une concentration de 0,2 à 5 mg par dose journalière et un ou des probiotiques étant utilisés à une concentration de 10e5 à 10e12 unités formant colonies (ufc) par dose journalière.

10. Myo-inositol et un ou plusieurs probiotiques pour une utilisation selon l'une quelconque des revendications 7 à 9, dans lesquels la vitamine B2, si elle est utilisée, est utilisée à une concentration de 0,14 à 14 mg par dose journalière, la vitamine B6, si elle est utilisée, étant utilisée à une concentration de 0,26 à 26 µg par dose journalière, la vitamine D, si elle est utilisée, étant utilisée à une concentration de 1,5 à 100 µg par dose journalière

11. Myo-inositol et un ou plusieurs probiotiques pour une utilisation selon l'une quelconque des revendications 7 à 10 dans lesquels le sujet étant un mammifère choisi dans le groupe constitué par ; un chat, un chien, un être humain, et ledit myo-inositol étant de préférence administré par voie entérale, plus préférablement par voie orale et de préférence sous la forme d'un produit nutritionnel, d'un produit alimentaire, d'un produit alimentaire fonctionnel, d'un produit pour un vieillissement en bonne santé, d'un produit laitier, d'un complément nutritionnel, d'une formulation pharmaceutique, d'un produit de boisson, d'un régime alimentaire ou d'un produit alimentaire pour animal de compagnie.
